# EUROPEAN PATENT SPECIFICATION

(11) **EP 1 266 161 B1**
(45) Date of publication and mention of the grant of the patent: **27.04.2005**
(21) Application number: 01908528.1
(22) Date of filing: 16.02.2001
(51) Int. Cl.: F16K 11/044

(54) **A METHOD AND A DEVICE FOR REDUCING GROWTH OF BACTERIA IN A WATER-MIXER WITH AN APPERTAINING PIPELINE**
VERFAHREN UND VORRICHTUNG ZUR VERMINDERUNG DES WACHSTUMS VON BAKTERIEN IN EINEM WASSERMISCHER UND IN EINEM ZUGEHÖRIGEN ROHR
PROCEDE ET DISPOSITIF DE REDUCTION DE LA CROISSANCE DE BACTERIES DANS UN MELANGEUR D'EAU ET DANS UNE CONDUITE EN DEPENDANT

(30) Priority: 17.02.2000 SE 0000515
(43) Date of publication of application: 18.12.2002
(73) Proprietor: FM MATTSSON AB, S-792 27 Mora (SE)
(72) Inventor: ANDERSSON, Sven, S-792 97 Mora (SE); ERICSSON, Stefan, S-792 37 Mora (SE)
(74) Representative: Estreen, Lars
(86) International application number: PCT/SE2001/000342
(87) International publication number: WO 2001/061224

(56) References cited:
- US-A- 5 050 640

## Description

### Field of invention

The present invention relates to a method for reducing the growth of bacteria in a water-mixer to which there is connected a warm water supply line and a cold water supply line and which includes a mixed water supply line to which there is fitted a shower nozzle, for instance a microphone-shaped nozzle.

The invention also relates to a thermostat mixer in a device of the aforesaid kind.

The bacterium legionella pneumophila can be found in all fresh water, particularly in surface water. The bacterium is not particularly dangerous in the small quantities in which it can be found in cold water. Colonisation of this bacterium is most significant at temperatures of about 40°C, i.e. showering temperatures. The growth of this colonisation is large enough to set people at risk of infection after only a few hours. This problem is not only found in shower mixers, but in all places where water is tapped, i.e. also in dish washer mixers and wash basin mixers for instance. Growth zones are predominantly edges, bio films and irregularities in water conduits and valves. The greatest growth risk, however, is found in static water at a temperature of about 40°C.

Flushing of the mixer with scalding water with a temperature of 80-85°C will kill the bacteria, so as to eliminate growth risk. However, flushing with scalding hot water constitutes, in itself, a serious problem, particularly in the case of shower mixers.

### Background of the invention

SE-C-510 400 (Nutsos) (Swedish Patent Application 9801626-4) proposes a solution to the problem caused by legionella pneumophila in mixer valves in connection with faucet installations. According to this proposal, the mixer shall be fitted with an additional warm water outlet from its warm water space, wherein the warm water outlet is adapted to be connected to a warm water return line through the medium of a second valve means in the closed mixer position, and to allow warm water to circulate through the warm water space in said closed mixer position. This document, however, would appear to disregard the fact that warmth will also be spread in the cold water conduit so as to create in this conduit a zone with a water temperature of 40-45°C, i.e. promote considerable risk of the growth of said bacterium.

A further example of the present standpoint of techniques is found in US,A, 5 069 241 (Hochstrasser), in which there is described a mixer valve that includes a two-sided valve element. When the water is turned off, the valve element is moved to an inoperative position by the force of two mutually counteracting springs. The conduit leading to the shower is therewith emptied automatically. However, the risk of legionella growth still remains in the actual mixer.

US,A, 5 050 640 (Cowley) describes a water mixing device according to the preamble of Claim I and Claim 6. This device is fitted with a hot water and cold water mixing valve (11). After turning off a conduit leading to the shower nozzle, the cold water conduit is cut-off and hot water is re-cycled via a short circuiting line to prevent the growth of bacteria. Alternatively, the process may be controlled by a sensor fitted to the means for holding the shower handle.

In the case of this known arrangement, the short circuiting line is in the actual mixing zone and bacterial growth can only be prevented with the use of hot water, which restricts the use options.

### Object of the invention

One object of the present invention is to provide a method and a device with which the serious problem of the growth of legionella pneumophila can be solved comparatively easily and reliably.

Another object is to provide a method and a device which provide alternative application possibilities in response to different prevailing conditions.

Yet another object of the invention is to provide a method and a device in which the various stages in the process can be controlled by a simple computer equipped with a circuit board and capable of functioning automatically, i.e. in the absence of human manipulation.

### Summary of the invention

These and other object of the invention are achieved by an inventive method that comprises the features set forth in the characterising clause of Claim 1.

The invention is based on the realisation that the growth of legionella pneumophila can be effectively counteracted by flushing hot water through the mixer and the associated mixed water supply conduit, wherein the temperature of the hot water differs considerably from the temperature zone at which the growth of such bacteria is at its greatest, e.g. a temperature zone in the region of 35-40°C. Thus, flushing of these elements can be effected either with hot or with cold water. Whether hot or cold water is used will depend greatly on the circumstances that prevail at the user location.

It is normally preferred to flush through the aforesaid elements with cold water, although water taken from the hot water supply conduit can be used instead to this end if insufficient cold water is available, as is often the case in tropical countries for instance.

Another advantage is that the bacteria killing effect achieved by this flushing action can be made more effective by adding a bactericidal chemical to the water. This can be readily achieved by connecting a conduit from a bactericide-containing container to one of the supply conduits to the mixer.

An important feature of the invention is that flushing of the aforesaid elements is stopped subsequent to having reached a desired state in the mixer and/or the consumer supply conduit.

This can be achieved, for instance, with the aid of a timer that delivers an impulse for stopping flushing of said elements and return to a normal state when flushing has taken place over a period of time that ensures that bacterial growth has ceased. This time period can be determined experimentally in each individual case. A more or less continuous flushing action, as suggested in the aforesaid Swedish document, is therefore unnecessary.

However, a sensor means, for instance a temperature sensor that detects the temperature in the mixer and/or in the shower supply conduit can be used instead of a timer, said temperature sensor automatically initiating a new flushing process when its detects a temperature that constitutes the risk of bacterial growth.

In a preferred method of application, the short circuiting line situated upstream of the mixing zone of the mixer includes a cut-off device, for instance a solenoid valve, that opens when a cut-off device, e.g. a solenoid valve, in the other conduit closes. Both of these cut-off devices, e.g. solenoid valves, can be controlled by the simple computer associated with the arrangement.

The invention also relates to an arrangement pertaining to water mixers for preventing the growth of bacteria of the aforementioned kind. The essential characteristic features of this arrangement are set forth in Claim 6. Advantageous embodiments of the arrangement are the subject of Claims 7-10.

Particularly when hot water is used as a flushing medium there is preferably used a pressure-controlled thermostat mixer, in order to avoid the risk of scalding the user. Naturally, a pressure-controlled thermostat mixer may also be used when flushing is effected with cold water, as is normally preferred.

The invention will now be described in more detail with reference to an exemplifying embodiment thereof and also with reference to the accompanying drawings.

Figure I is a schematic perspective view of a mixer arrangement according to the invention.

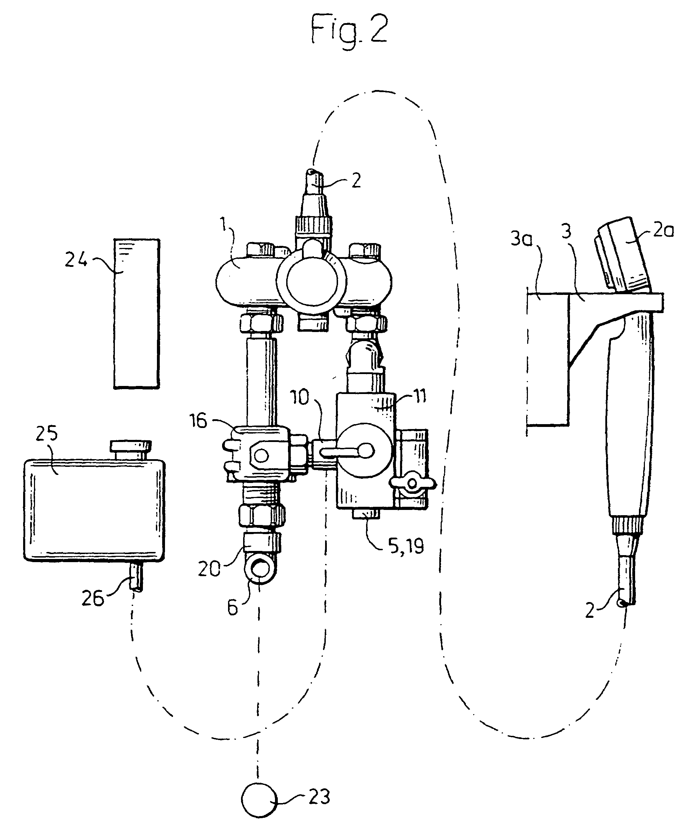

Figure 2 illustrates the mixer arrangement schematically, although in a somewhat more detailed front view in relation to Figure 1.

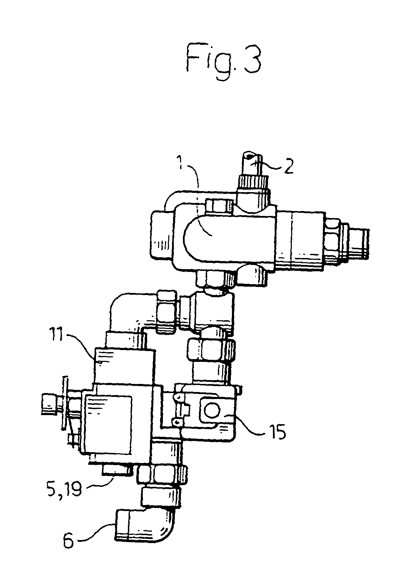

Figure 3 is a side view of some of the elements included in the arrangement shown in Figure 2.

### Description of preferred embodiments

Shown in the drawings is a mixer 1 for mixing hot and cold water, said mixer being provided conventionally with means for opening/closing the mixer and for adjusting the temperature of the mixed water.

The mixer may, for instance, be a mechanical mixer, a single grip mixer, or a thermostat mixer which may be pressure-controlled, as is particularly suitable if the risk of scalding is to be avoided.

Connected to the mixer 1 is a shower supply conduit or consumer supply conduit 2 which, in the illustrated case, is fitted with a shower nozzle, a so-called microphone-shaped nozzle 2a. A cold water supply conduit 5 and a hot water supply conduit 6 are connected to the mixer. A holder 3 on which the shower nozzle can be hung or placed includes an electric sensor element which detects when the shower nozzle is placed on the holder after showering. Other types of sensor elements may be used, for instance a movement detector that registers when the shower nozzle is placed on the holder, or when the person using the shower leaves the shower space.

The cold water supply conduit 5 includes a switching element, in the illustrated case a solenoid valve 15 with servo motor 11. Located between the cold water supply conduit 5 and the hot water supply conduit 5 upstream of the mixing zone of the mixer is a short circuiting conduit 10. The switching device, i.e. the servo motor 1I and solenoid valve 15, is adapted to place the cold water supply conduit 5 in communication with the hot water supply conduit 6 in response to an impulse. As a result, the solenoid valve 15 in the short circuiting conduit 10 is opened and a solenoid valve 16 in the hot water supply conduit 6 is closed, so as to flush the mixer I and the shower nozzle supply conduit 2 with cold water.

In order to achieve a reliable function of the flushing process, the cold water supply conduit 5 includes a check valve 19 upstream of the servo motor 11, and the hot water supply conduit 6 includes a check valve 20 upstream of the solenoid valve 16.

The mixer 1 and/or the shower nozzle supply conduit includes a device for detecting a desired state in said mixer and/or in said nozzle supply conduit. The desired state is normally one in which there is no longer any risk of bacterial growth in the aforesaid elements. When this state is reached, the device sends an impulse to a computer 24 that forms part of said arrangement and that includes a circuit board for controlling the flushing process and the various functions of the arrangement.

The computer controls the continuation of the flushing process, by switching-off the solenoid valve 15 in the short circuiting conduit 10 and also a further solenoid valve (not shown) in the cold water supply conduit. The cut-off valve 1b on the mixer I need not therefore be actuated in this case.

The device used to cut-off the flushing process is suitably a sensor element, e.g. a temperature sensor 22 which detects the temperature in the mixer I and/or in the shower nozzle supply conduit 2. If this temperature should approach the critical value of about 35-42°C, where the danger of bacterial growth exists, a new flushing process with cold water is commenced. This second flushing process is also terminated when a sufficiently low temperature has been registered in both of said elements in order to avoid the danger of bacterial growth (at least temporarily).

Although not shown, further sensors may be provided at different places in the hot water supply and/or the cold water supply conduits, particularly when these conduits are insufficiently de-insulated and pass, for instance, heated spaces where there is a risk of the particular temperature in the cold water supply conduit becoming too high if no flushing is undertaken.

In an alternative application of the invention, flushing is effected in a corresponding manner but with hot water instead. The course followed by the flushing process and the components involved are essentially the same as those in the aforegoing, although the supply conduit 5 and 6 for cold and hot water respectively shall change places. Expressed in another way, the drawing shall be seen in mirror image about a centre plane through the illustrated arrangement.

When flushing is effected with hot water, the water will preferably have a high temperature, e.g. a temperature in the range of 80-85°C, at which all of the bacteria present in the system will be killed. When the temperature in the mixer and/or in the shower nozzle supply conduit, or consumer supply conduit, falls, renewed flushing of said elements with hot water must be undertaken, optionally time controlled.

In certain cases, particularly when the arrangement is used in hot climates, possibly in tropical countries, where sufficiently low temperatures of the water in the cold water supply conduit cannot be guaranteed, the arrangement may conveniently be adapted to co-act with a system for delivering a bactericidal chemical substance. When flushing is effected with hot water, the bactericidal agent is introduced into the cold water supply conduit over a distance therein in which the spread of heat can be expected to take place.

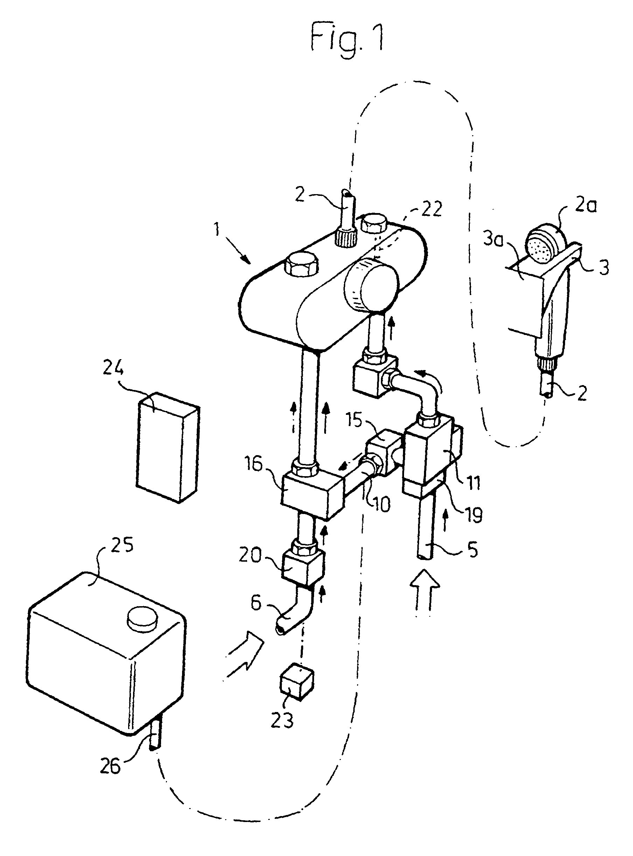

Thus, the arrangement is shown in Figure 1 to include a container 25 for such a bactericidal agent. The container is connected to one of the conduits in the flushing system by a conduit 26 shown in a chain line, for supplying said agent in conjunction with the flushing process. The container 25 may be situated at a higher level than the mixer 1 and may include a solenoid valve (not shown) which is controlled by the computer 2 to open said valve concurrently with the flushing process. Alternatively, the container may be equipped with a pump (not shown) that is started-up concurrently with said flushing process.

The bactericidal chemical agent may be supplied to the system regardless of whether cold water or hot water is used in conjunction with the flushing process. The cold water need not therewith have as low a temperature or the hot water as high a temperature as would otherwise be the case, i.e. when flushing of said elements is effected in the absence of a chemical agent. In actual fact, flushing may sometimes be effected at temperatures that lie close to the range in which the risk of bacterial growth can be judged to be serious in the absence of such an agent, i.e. a temperature range of 35-42°C.

By "cold water" as used in the aforegoing is meant water whose temperature is beneath 20°C, whereas by "hot water" is meant water whose temperature is above 60-65°C. However, in order to ensure that all bacteria present will be killed, the hot water will preferably have a temperature in the range of 70-75°C or higher.

It will be understood that the invention can be varied in different ways within the scope of the inventive concept as defined in the accompanying claims.

## Claims

1. A method of reducing the growth of bacteria in a water-mixer and affiliated mixed-water consumer supply conduit (2) connected for instance to a shower nozzle (2a) such as a so-called microphone-shaped nozzle, wherein the mixer (1) is connected to a cold water supply conduit (5) and to a hot water supply conduit (6), wherein termination of a water consumption process, e.g. a showering process, is detected with the aid of suitable means (3a) which sends an impulse to a switching element, e.g. a valve (15), wherein one of the supply conduits (5; 6) for cold and hot water respectively is connected to the other via a short circuiting conduit (10) and, at the same time, closes the standard supply of water to the other conduit via another means, so that the mixer (1) and the shower supply conduit (2) will be flushed through with water from said one supply conduit, **characterised in that** the short circuiting conduit (10) is placed upstream of the mixing zone of the water mixer, and flushed with either cold water or hot water, whereby the flushing process is stopped when a desired state has been reached in the mixer and/or in the consumer supply conduit (2).

2. A method according to Claim 1, **characterised by** repeating the flushing process when detecting in the mixer (1) or in the shower supply conduit (2) a state in which there is a risk of bacterial growth.

3. A method according to Claim I or 2, **characterised by** delivering a chemical bactericide to one of said conduits during a flushing process.

4. A method according to any one of Claims 1-3, **characterised in that** the short circuiting conduit (10) includes a cut-off device, e.g. a solenoid valve (15) which opens when a cut-off device, e.g. a solenoid valve (16), in the other conduit closes.

5. A method according to any one of Claims 1-4, **characterised in that** the cold water supply conduit (5) is connected to the hot water supply conduit (6) via the short circuiting conduit (10) for flushing the mixer (1) and the consumer supply conduit (2) with cold water.

6. An arrangement relating to mixers for preventing the growth of bacteria therein and a consumer conduit (2) connected to the mixer (1) and provided preferably with a microphone-shaped shower nozzle (2a), wherein the mixer (1) is connected to a cold water supply conduit (5) and to a hot water supply conduit (6), and wherein the arrangement includes
a) a device (3a) for detecting the termination of a water consuming process,
b) a short circuiting conduit (10) between the two supply conduits (5; 6),
c) a switching element, e.g. a valve (15), for communication of one supply conduit with the other via the short circuiting conduit (10) in response to an impulse delivered from the detecting element (3a), such as to flush a mixing zone of the mixer (1) and the consumer conduit (2) with water from said one supply conduit,
d) valve means, e.g. a solenoid valve (15), and
e) means for delivering an impulse for cessation of the flushing process upon reaching a desired state in the mixer (1) and/or in the consumer conduit (2), **characterised in that** the short circuiting conduit (10) is located upstream of the mixing zone; **in that** the switching means, e.g. a valve (15) with servo motor (11), is located in one of said supply conduits (5; 6), and **in that** a valve means, e.g. a solenoid valve ( 14), is provided in the other supply conduit.

7. An arrangement according to Claim 6, **characterised in that** the device for stopping the flushing process is a timer.

8. An arrangement according to Claim 6 or 7, **characterised in that** a conduit (26) extending from a container (25) that contains a chemical bactericidal agent is connected to one of said supply conduits (5; 6; 10) for supplying said bactericidal agent concurrently with the flushing process.

9. An arrangement according to any one of Claims 6-8, **characterised in that** the cold water supply conduit (5) is adapted for communication with the hot water supply conduit (6) via said short circuiting conduit (10), for flushing the mixer (1) and the consumer conduit (2) with cold water.

10. An arrangement according to any one of Claim 6-9, **characterised in that** the detecting means and flushing regulating means (3a; 11; 15; 16; 22; 23) are connected to a computer (24) provided with a circuit board for controlling said flushing process.

## Patentansprüche

1. Ein Verfahren zum Reduzieren des Bakterienwachstums in einem Wassermischer und einer zugeordneten Mischwasser-Verbraucher-Zufuhrleitung (2), verbunden z.B. mit einer Duschdüse (2a) wie einer so genannten mikrofongeformten Düse, wobei der Mischer (1) mit einer Kaltwasser-Zufuhrleitung (5) und einer Heißwasser-Zufuhrleitung (6) verbunden ist, wobei die Beendigung eines Wasserverbrauchsvorgangs, z.B. eines Duschvorgangs, mit Hilfe einer geeigneten Einrichtung (3a) erfasst wird, welche einen Impuls zu einem Schaltelement, z.B. einem Ventil (15) sendet, wobei eine der Zufuhrleitungen (5; 6) jeweils für Kalt- und Heißwasser mit der anderen über eine Kurzschlussleitung (10) verbunden ist und zur gleichen Zeit die Standard-Wasserzufuhr zu der anderen Leitung über eine andere Einrichtung schließt, so dass der Mischer (1) und die Dusch-Zufuhrleitung (2) durchgespült werden mit Wasser von der einen Zufuhrleitung, **dadurch gekennzeichnet, dass** die Kurzschlussleitung (10) stromaufwärts vom Mischbereich des Wassermischers angeordnet ist und entweder mit Kaltwasser oder Heißwasser gespült wird, wobei der Spülvorgang angehalten wird, wenn ein gewünschter Zustand im Mischer und/oder in der Verbraucher-Zufuhrleitung (2) erreicht worden ist.

2. Verfahren nach Anspruch 1, **gekennzeichnet durch** Wiederholen des Spülvorganges, wenn in dem Mischer (1) oder der Dusch-Zufuhrleitung (2) ein Zustand erfasst wird, in welchem ein Risiko von Bakterienwachstum vorliegt.

3. Verfahren nach Anspruch 1 oder 2, **gekennzeichnet durch** Liefern eines chemischen Bakterizids zu einer der Leitungen während eines Spülvorgangs.

4. Verfahren nach einem der Ansprüche 1 bis 3, **dadurch gekennzeichnet, dass** die Kurzschlussleitung (10) eine Abschaltvorrichtung, z.B. ein Solenoidventil (15) aufweist, welches sich öffnet, wenn sich eine Abschaltvorrichtung, z.B. ein Solenoidventil (16), in der anderen Leitung schließt.

5. Verfahren nach einem der Ansprüche 1 bis 4, **dadurch gekennzeichnet, dass** die Kaltwasser-Zufuhrleitung (5) mit der Heißwasser-Zufuhrleitung (6) über die Kurschlussleitung (10) verbunden ist zum Spülen des Mischers (1) und der Verbraucher-Zufuhrleitung (2) mit Kaltwasser.

6. Eine Anordnung in Bezug auf Mischer zum Verhindern des Bakterienwachstums in diesen und auf eine Verbraucherleitung (2), verbunden mit dem Mischer (1) und vorzugsweise vorgesehen mit einer mikrofongeformten Duschdüse (2a), wobei der Mischer (1) mit einer Kaltwasser-Zufuhrleitung (5) und einer Heißwasser-Zufuhrleitung (6) verbunden ist, und wobei die Anordnung aufweist
a) eine Vorrichtung (3a) zum Erfassen der Beendigung eines Wasserverbrauchsvorgangs,
b) eine Kurzschlussleitung (10) zwischen den beiden Zufuhrleitungen (5; 6),
c) ein Schaltelement, z.B. ein Ventil (15), zur Kommunikation einer Zufuhrleitung mit der anderen über die Kurzschlussleitung (10) in Antwort auf einen Impuls, geliefert von dem Erfassungselement (3a), um einen Mischbereich des Mischers (1) und die Verbraucherleitung (2) mit Wasser von der einen Zufuhrleitung zu spülen,
d) eine Ventileinrichtung, z.B. ein Solenoidventil (15), und
e) eine Einrichtung zum Liefern eines Impulses zur Beendigung des Spülvorgangs nach Erreichen eines gewünschten Zustandes im Mischer (1) und/oder in der Verbraucherleitung (2), **dadurch gekennzeichnet, dass** die Kurzschlussleitung (10) stromaufwärts von dem Mischbereich angeordnet ist, dass die Schalteinrichtung, z.B. ein Ventil (15) mit Servomotor (11), in einer der Zufuhrleitungen (5; 6) angeordnet ist und dass eine Ventileinrichtung, z.B. ein Solenoidventil (16), in der anderen Zufuhrleitung vorgesehen ist.

7. Anordnung nach Anspruch 6, **dadurch gekennzeichnet, dass** die Vorrichtung zum Anhalten des Spülvorganges ein Zeitgeber ist.

8. Anordnung nach Anspruch 6 oder 7, **dadurch gekennzeichnet, dass** eine Leitung (26), die sich von einem Behälter (25) aus erstreckt, der ein chemisches Bakterizidmittel enthält, mit einer der Zufuhrleitungen (5; 6; 10) verbunden ist zum Zuführen des Bakterizidmittels gleichzeitig mit dem Spülvorgang.

9. Anordnung nach einem der Ansprüche 6 bis 8, **dadurch gekennzeichnet, dass** die Kaltwasser-Zufuhrleitung (5) geeignet ist für die Kommunikation mit der Heißwasser-Zufuhrleitung (6) über die Kurzschlussleitung (10) zum Spülen des Mischers (1) und der Verbraucherleitung (2) mit Kaltwasser.

10. Anordnung nach einem der Ansprüche 6 bis 9, **dadurch gekennzeichnet, dass** die Erfassungseinrichtung und die Spülreguliereinrichtung (3a; 11; 15; 16; 22; 23) mit einem Computer (24) verbunden sind, der mit einer Schalttafel zum Kontrollieren des Spülvorgangs versehen ist.

## Revendications

1. Procédé de réduction de la croissance des bactéries dans un mélangeur d'eau et une canalisation d'alimentation du consommateur d'une eau résultante d'un mélange d'eaux associée (2) reliés par exemple à une buse de douche (2a) comme ce qu'on appelle une buse en forme de microphone, dans lequel le mélangeur (1) est relié à une canalisation d'alimentation de l'eau froide (5) et à une canalisation d'alimentation de l'eau chaude (6), dans lequel la fin d'un processus de consommation de l'eau, par exemple un processus de douche, est détecté à l'aide d'un moyen adapté (3a) qui envoie une impulsion à un élément de commutation, par exemple une valve (15), dans lequel une des canalisations d'alimentation (5 ; 6) pour l'eau froide et l'eau chaude respectivement est reliée à l'autre par une canalisation de court-circuit (10) et, au même moment, ferme l'alimentation standard en eau vers l'autre canalisation par un autre moyen, de sorte que le mélangeur (1) et la canalisation d'alimentation de la douche (2) soient rincés par de l'eau provenant de ladite une canalisation d'alimentation, **caractérisé en ce que** la canalisation de court-circuit (10) est placée en amont de la zone de mélange du mélangeur d'eau, et rincée avec de l'eau froide ou de l'eau chaude, de sorte que le processus de rinçage soit stoppé quand un état souhaité a été atteint dans le mélangeur et/ou dans la canalisation d'alimentation du consommateur (2).

2. Procédé selon la revendication 1, **caractérisé par** la répétition du processus de rinçage lors de la détection dans le mélangeur (1) ou dans la canalisation d'alimentation de la douche (2) d'un état dans lequel il y a un risque de croissance bactérienne.

3. Procédé selon la revendication 1 ou 2, **caractérisé par** la délivrance d'un bactéricide chimique à l'une desdites canalisations au cours du processus de rinçage.

4. Procédé selon l'une quelconque des revendications 1 à 3, **caractérisé en ce que** la canalisation de court-circuit (10) comprend un dispositif d'interruption, par exemple une électrovanne (15) qui s'ouvre quand un dispositif d'interruption, par exemple une électrovanne (16), se ferme dans l'autre canalisation.

5. Procédé selon l'une quelconque des revendications 1 à 4, **caractérisé en ce que** la canalisation d'alimentation de l'eau froide (5) est reliée à la canalisation d'alimentation de l'eau chaude (6) par la canalisation de court-circuit (10) pour rincer le mélangeur (1) et la canalisation d'alimentation du consommateur (2) avec de l'eau froide.

6. Agencement concernant les mélangeurs pour y empêcher la croissance des bactéries et une canalisation de consommateur (2) reliée au mélangeur (1) et dotée de préférence d'une buse de douche en forme de microphone (2a), dans laquelle le mélangeur (1) est relié à une canalisation d'alimentation d'eau froide (5) et à une canalisation d'alimentation de l'eau chaude (6) et/ou la disposition comprend
a) un dispositif (3a) pour détecter la fin d'un processus de consommation de l'eau,
b) une canalisation de court-circuit (10) entre les deux canalisations d'alimentation (5 ; 6),
c) un élément de commutation, par exemple une valve (15), pour la communication d'une canalisation d'alimentation avec l'autre par le conduit de court-circuit (10) en réponse à une impulsion délivrée par l'élément de détection (3a), de sorte à rincer une zone de mélange du mélangeur (1) et la canalisation du consommateur (2) avec l'eau depuis ladite une canalisation d'alimentation,
d) un moyen de valve, par exemple une électrovanne (15), et
e) un moyen pour délivrer une impulsion pour l'arrêt du processus de rinçage une fois un état souhaité atteint dans le mélangeur (1) et/ou dans la canalisation du consommateur (2), **caractérisée en ce que** la canalisation de court-circuit (10) est située en amont de la zone de mélange ; **en ce que** le moyen de commutation, par exemple une valve (15) avec un cerveau moteur (11), est situé dans l'une desdites canalisations d'alimentation (5 ; 6), et **en ce que** un moyen de valve, par exemple une électrovanne (14), est fournie dans l'autre canalisation d'alimentation.

7. Agencement selon la revendication 6, **caractérisée en ce que** le dispositif pour stopper le processus de rinçage est une minuterie.

8. Agencement selon la revendication 6 ou 7, **caractérisée en ce qu'**une canalisation (26) s'étendant depuis un récipient (25) qui contient un agent bactéricide chimique est relié à l'une desdites canalisations d'alimentation (5 ; 6 ; 10) pour fournir ledit agent bactéricide simultanément avec le processus de rinçage.

9. Agencement selon l'une quelconque des revendications 6 à 8, **caractérisée en ce que** la canalisation d'alimentation de l'eau froide (5) est adaptée pour la communication avec la canalisation d'alimentation de l'eau chaude (6) par ladite canalisation de court-circuit (10), pour rincer le mélangeur (1) et la canalisation du consommateur (2) avec l'eau froide.

10. Agencement selon l'une quelconque des revendications 6 à 9, **caractérisée en ce que** le moyen de détection et le moyen de régulation du rinçage (3a ; 11 ; 15 ; 16 ; 22 ; 23) sont reliés à un ordinateur (24) doté de cartes de circuit imprimé pour contrôler ledit processus de rinçage.
